Europäisches Patentamt

European Patent Office

Office européen des brevets

(11) Publication number : **0 365 087 B1**

# EUROPEAN PATENT SPECIFICATION

(45) Date of publication of patent specification :
**22.07.92 Bulletin 92/30**

(51) Int. Cl.[5] : **A61K 47/48**

(21) Application number : **89202588.3**

(22) Date of filing : **13.10.89**

(54) **Immunotoxins for the treatment or prophylaxis of auto-immune diseases.**

(30) Priority : **21.10.88 EP 88202349**

(43) Date of publication of application :
**25.04.90 Bulletin 90/17**

(45) Publication of the grant of the patent :
**22.07.92 Bulletin 92/30**

(84) Designated Contracting States :
**AT BE CH DE ES FR GB GR IT LI NL SE**

(56) References cited :
**EP-A- 0 262 710**
**DE-A- 3 513 572**
**GB-A- 2 146 338**

(56) References cited :
**THE JOURNAL OF IMMUNOLOGY, vol. 135, no.
5, November 1985, pages 3062-3067, The
American Association of Immunologists,Bal-
timore, US; C.A. OLSBERG et al.: "Selective in
vitro inhibition of an antibody response to
purified acetylcholine receptor byusing anti-
gen-ricin a chain immunotoxin"**

(73) Proprietor : **AKZO N.V.**
**Velperweg 76**
**NL-6824 BM Arnhem (NL)**

(72) Inventor : **Verheul, Hermanus Antonius Maria**
**Van Sonsbeeckstraat 10**
**NL-5344 JB Oss (NL)**

(74) Representative : **Hermans, Franciscus G.M. et
al**
**Patent Department AKZO N.V. Pharma
Division P.O. Box 20**
**NL-5340 BH Oss (NL)**

## Description

The present invention is concerned with immunotoxins for the treatment or prophylaxis of an auto-immune disease, pharmaceutical preparations containing one or more immunotoxins and a process for the preparation of said immunotoxins.

The immune system displays a complexity, which includes a requirement for self-recognition. Regulation of the immune system occurs through this self-recognition and may involve cells, antibodies, amplification systems (e.g. complement) and combinations of these elements. In healthy individuals the immune system will respond to exposure to a foreign substance by activating the cellular immune response and/or by producing specific antibodies against this substance. On the other hand the immune system is generally tolerant to constituents of the own body, the so called self-tolerance. Auto-immunity may be defined as a circumvention of this self-tolerance.

Auto-immune diseases may be multiple sclerosis, in which the tissue attacked is myelin; myasthenia gravis, in which the target is a receptor molecule for the important neurotransmitter acetylcholine; rheumatoid arthritis, in which inter alia the peripheral joints are targeted, type I diabetes mellitus, characterized in that the cells producing insulin are destroyed and systemic lupus erythematosus, in which blood vessels, skin and kidney are attacked.

Auto-immune diseases can be initiated without the requirement for a specific external agent. The loss of tolerance to the self-antigens can be caused by the self-antigen itself, by an abnormal immune response to the self-antigen or by a combination of both. Some times exogenous agents provoke an immune response, which includes responsiveness to certain self-antigens (epitope mimicry). An immune response mounted by the host against a specific determinant of an infecting agent may cross-react with the mimicked host sequence, leading to auto-immunity and possible tissue injury and disease. Many viral and bacterial proteins share epitopes with host cell proteins that are similar enough to cross-react, yet different enough to break immunological tolerance.

The recognition of the self-antigens and/or exogenous antigens and the subsequently occurring damage of the self-antigen containing tissue by the immune system is mediated by the T-lymphocytes and B-lymphocytes.

In US patent 4,634,590 culturing of specific T-lymphocytes, responsible for causing auto-immune diseases in laboratory animals, as long term cell lines is described. It was found that after attenuation of these specific T-lymphocytes, the cells or membrane material thereof can be used as effective agents for vaccination against a specific auto-immune disease.

WO 85/05034 describes the use of a Mycobacterium or a fraction thereof in a composition or vaccin for alleviation of the symptoms or for the treatment or diagnosis of arthritic diseases. Specific fractions of Mycobacterium, which show immunological cross-reactivity with proteoglycans of normal joint cartilage are identified. Protection against adjuvant arthritis (AA) and suppression of the auto-immune disease can be associated with one specific fraction of Mycobacterium, while the other fraction induces the harmful auto-immune response.

Furthermore two T-lymphocyte clones were identified responding to the two Mycobacterium fractions respectively, thus can be associated with arthritogenicity or with suppression of arthritis.

Adjuvant Arthritis T-lymphocyte clones were also used to identify the antigen present on Mycobacterium recognized by these T-cell clones. A 65 kD protein was identified as the Mycobacterium antigen eliciting proliferative response of the T-cell clones in vitro. The epitope recognized by the T-cell clones resides in the 180-188 amino acid sequence of the 65 kD antigen. This sequence shows a similarity with a part of the link protein of rat proteoglycan (see van Eden et al., Nature 331, 171, 1988).

Current therapeutic treatment of auto-immune diseases includes the administration of non-specific drugs suppressing the auto-immune response but also producing various undesirable complications associated with the total depression of the immune system or causing other unwanted toxic side-effects to non-lymphoid tissues. Examples of drugs currently used combating auto-immune diseases are naproxen, auranofin, penicillamine, chloroquine and corticosteroids. Thus in a preferable approach for the treatment or prophylaxis of an auto-immune disease, drugs have to be targeted in a way that specifically affects the immunologic reactivity leading to the disease symptoms.

It is known from the literature that the acetylcholine receptor, being the self-antigen under attack in the auto-immune disease myasthenia gravis, can be used for suppression of the specific immune response against the antigen. The acetylcholine receptor linked to the toxins ricin or gelonin is able to eliminate a fraction of the antigen-reactive lymphocytes (Killen, J.A. and Lindstrom, J.M., Journal of Immunology 133 (1984), 2549-2553; Olsberg, C.A. et al., Journal of Immunology 135 (1985), 3062-3067; Brust, S. et al., Biol. Chem. Hoppe-Seyler 368 (1987), 991-999).

It is further known that an antigen in most cases can only be recognized by T-lymphocytes after processing of said antigen by antigen presenting cells (APC) in combination with molecules of the major histocompatibility complex (MHC). Consequently, the above-noted prior art antigen-toxin approach has the disadvantage that during APC processing of the antigen-toxin conjugate APC become inactivated by action of the toxin, depriving the immune system of valuable cells.

The present invention resides in the fact that specific self epitopes or exogenous epitopes are being conjugated to a cytotoxic substance instead of the entire self antigen or exogenous antigen, circumventing the necessity of processing by APC.

Furthermore, in the present situation the cytotoxic substance is now linked to that fragment of the antigen which is directly recognized by the T-lymphocyte: the epitope. This results in a more efficient toxic action of the epitope-cytotoxic substance conjugate for the T-lymphocytes allowing the use of reduced concentration of said epitope-cytotoxic substance conjugate in order to achieve complete elimination of auto-immunogenic T-lymphocytes.

Moreover, in the case of exogenous antigens, in the present invention only those T-lymphocytes responsible for the auto-immune response are eliminated allowing the immune system to evoke an immune response against the exogenous antigen.

The immunotoxin according to the present invention is thus characterized in that the immunotoxin comprises an epitope, recognized by T-lymphocytes being characteristic for the specific auto-immune disease or a cross-reactive analogue thereof, said epitope or cross-reactive analogue thereof being coupled to a cytotoxic substance.

Many proteins and/or epitopes that are recognized by T-lymphocytes being characteristic for the specific auto-immune disease are known and described in the literature.

Adjuvant Arthritis (AA) is a chronic auto-immune disease which causes the degeneration of the cartilage in the joints. Adjuvant Arthritis can be induced in rats by immunization with an antigen of Mycobacterium tuberculosis (Mt). The tissue damage seen in AA is characteristic of T-lymphocytes. The T-lymphocytes are stimulated by an antigen belonging to Mycobacterium tuberculosis which mimics the self antigens that are under attack in the arthritic rats.

Specific T-lymphocyte clones have been found that are able to recognize both the foreign Mycobacterium tuberculosis antigen and the self antigen. The self antigen probably resides within a part of a cartilage proteoglycan molecule, called the link protein. These specific T-lymphocyte clones react specifically with a 65 kD protein, a Mt protein expressed by E. coli transfected with Mt DNA. This 65 kD protein plays an important role in the immune response to Mycobacterium strains.

A peptide consisting of 21 amino acids residing in the 65-85 amino acid sequence of the 65 kD protein as well as a peptide consisting of nine amino acids, Thr-Phe-Gly-Leu-Gln-Leu-Glu-Leu-Thr, residing in the 180-188 amino acid sequence of the 65 kD protein are identified as the epitopes which are recognized by the T-lymphocytes. The said nonapeptide shows a resemblance with a part of the link protein of the proteoglycan molecule which joins the core protein to the sugar backbone of this molecule and has the amino acid sequence Thr-Ala-Val-Val-Ala-Leu-Glu-Leu-Gln. The 65 kD protein of Mycobacterium bovis which has an identical coding region as the Mt 65 kD protein contains also several epitopes which are recognized by lymphocytes.

These T-lymphocyte epitopes coincide or reside in the amino acid sequences 1-16, 17-61, 85-108, 112-132, 235-279 and 437-459 (for amino acid sequences of the Mycobacterium 65 kD protein see Thole et al., Infect. Immun. 55, 1466-1475, 1987; Husson et al., PNAS 84, 1679-1683, 1987; Shinnick et al., J.Bact. 169, 1080-1088, 1987).

Myelin basic protein (BP) is an auto-antigen in different species. Immunization with BP or passive transfer of lymphocytes from animals immunized with BP induces the auto-immune disease experimental auto-immune encephalomyelitis (EAE) in several species. EAE causes inflammatory paralysis and demyelination, symptoms also occurring with multiple sclerosis. EAE is mediated by a specific class of T-lymphocytes. Immunization with BP or some fragments thereof induces EAE in susceptable species, however not every fragment is encephalogenic. T-lymphocytes are only activated by the intact protein or fragments containing a specific epitope. Several epitopes within BP are already known in the art. The amino acid sequences 1-16, 1-37, 59-74, 68-88, 89-169 and 114-122 of BP have already been identified as epitopes or epitope containing sequences, showing affinity for encephalogenic T-lymphocytes and hence for T-lymphocytes characteristic for multiple sclerosis (for amino acid sequences of the myelin basic protein see Eylar et al., J.Biol.Chem. 246, 5770, 1971; Gibson et al., J.Biol.Chem. 259, 5028, 1984; Stoner, G.L. J.Neurochem. 43, 433-447, 1984; Scoble et al., J-.Neurochem. 47, 614-616, 1986).

Myasthenia gravis (MG), another auto-immune disease, is initiated by the presentation of an immunogenic factor of the acetylcholine receptor, resulting in a stimulation of auto-immune lymphocytes. MG is characterized by auto-antibodies and T-lymphocytes directed against the acetylcholine receptor resulting in increased degradation of the receptor and manifests itself clinically by weakness and fatigue of voluntary muscles. The acetylcholine receptor consists of 5 subunits ($\alpha 2\beta cd$).

A major part of the cellular auto-immune response of patients suffering from MG is directed to an area on the $\alpha$-subunit of the receptor: the main immunogenic region. In this region several epitopes or epitope containing amino acid sequences have been identified such as the amino acid sequences 1-30, 73-90, 100-116, 111-126, 146-162, 169-181, 182-198, 257-271, 351-368 (for amino acid sequences of the acetylcholine receptor see Noda et al., Nature 299, 793-797, 1982 and Nature 302, 528-532, 1983 and Nature 305, 818-823, 1983; Sumikawa et al., Nucleic Acid Res. 10, 5809-5822, 1982; Devillers-Thiery et al., PNAS 80, 2067-2071, 1983; Hohlfeld et al., J.Clin.Invest. 81, 657-660, 1988).

The term epitope as used herein denotes an immunogenic determinant of an immunogenic molecule, the immunogenic determinant being recognized by a component of the immune system.

Above-noted epitopes are candidates for coupling to a cytotoxic substance resulting in an epitope-cytotoxic substance conjugate according to the present invention.

It is obvious for a person skilled in the art that a peptide containing only a part of an epitope will also show a binding affinity for the T-lymphocytes as will be the case with a peptide containing in addition to the epitope flanking amino acids or other moieties. The length of these flanking moieties is limited by the requirement that the peptide is not necessarily processed by APC. It is clear that an immunotoxin comprising such peptides is also within the scope of this invention.

Furthermore, it is possible that polypeptides having a similar but not identical amino acid sequence show corresponding immunologic characteristics. Polypeptides related to an epitope in the above-noted way are also included in the present invention.

Cytotoxic substances to be used in the framework of the present invention are selected from a group of substances which are able to stop cell proliferation and eventually cause death of the cell.

Preferably the cytotoxic substances are selected from a group of proteins of plant, fungal or bacterial origin, having the property of inhibiting protein synthesis in eukaryotic cells. These toxins specifically act on the process of translation by catalytically inactivating the ribosomes. The plant toxins are classified in two groups i.e. toxins composed of either two or four protein-containing subunits and toxins consisting of a single chain protein molecule to which a carbohydrate moiety is usually linked.

The two-subunit toxins have a molecular mass of 60-65 kD, subunit A being slightly smaller than the B subunit (30 kD vs 32 kD). The subunits are covalently linked by a single disulphide bond, but hydrophobic interactions are also relevant to keep A and B together. Subunit B consists of a polypeptide chain and a carbohydrate chain that contains mannose and N-Ac-glucosamine. This subunit appears to be involved in the interaction of the toxin with the galactose-containing glycoproteins and -lipids of the target cell membrane. This subunit allows the A subunit to enter into the cell. Subunit A inactivates eukaryotic ribosomes in a catalytic way, with consequent inhibition of protein synthesis and death of the cell. Included in this group of toxins are ricin, abrin, modeccin, volkensin and viscumin.

The single chain plant toxins have properties similar to those of the A subunits described above. They are inactive upon intact cells although they are very potent inhibitors in cell-free systems. Because of the absence of a B subunit these toxins enter with difficulty into the target cells, thus being much less toxic to cells than the B subunit containing toxins. However, they can become toxic if they are linked to carriers capable of binding to or entering cells such as lectins and antibodies. The following proteins belong to this class of toxins, e.g. gelonin, pokeweed antiviral proteins, dodecandrin, dianthins, luffin, saporins, Momardica charantia inhibitor, grain inhibitors.

The fungal protein toxins, e.g.: $\alpha$-Sarcin, restrictocin and mitogillin are closely related basic proteins. These toxins contain a single polypeptide chain and unlike most of the plant toxins do not contain a carbohydrate moiety.

Diphtheria toxin and Pseudomonas exotoxin are two examples of bacterial toxins that show functional similarities with the above described 2 subunit plant toxins.

Other prefered cytotoxic substances are selected from the group of radio-isotopes. These radio-isotopes can be targeted to specific cells. The target cells are damaged or killed by the use of isotopes that emit energetic $\alpha$-particles or $\beta$-particles. Suitable isotopes are $^{90}$Y, $^{153}$Sm, $^{43}$Sc, $^{67}$Cu, $^{186}$Rh, $^{188}$Rh, $^{212}$Bi, $^{211}$At, $^{103}$Pd.

There are a number of well known ways to conjugate an epitope to a protein toxin.

Random coupling between the epitope and a protein toxin can for example be achieved by using a carbodiimide compound (e.g. EDC) that activates the carboxyl group of a protein toxin so that it may be coupled to the amino group of the epitope.

Apart from this direct coupling linkers can be used to provide a bridge between the epitope and a protein toxin. A well known homobifunctional crosslinking agent is glutar(di)aldehyde. It reacts preferentially with amines.

Other homobifunctional linkers can be selected from the group of imido-esters (e.g. dimethyl adipimidate, dimethyl suberimidate) or N-hydroxysuccinimide esters (e.g. disuccinimidyl suberate, dithiobis(succinimidyl propionate), both having specifity for amine groups.

Heterobifunctional reagents are preferred since they contain two dissimilar functional groups that allow control of the conjugation reaction both selectively and sequentially. An example of an heterobifunctional linker is N-succinimidyl-3(2-pyridyldithio)propionate (SPDP). First a 2-pyridyl-disulphide group is introduced into the epitope by reacting a primary amine with SPDP. The derivatised epitope can now be conjugated to a protein toxin containing a thiol group, forming a disulphide bond between the epitope and protein toxin. If not present already this thiol group can be introduced into the protein toxin by thiolating agents such as 2-iminothiolane, SPDP or N-succinimidyl-S-acetylthioacetate.

Maleimide groups as well as bromo- or iodoacetyl groups react under mild conditions fairly rapidly with thiol groups to give a thio-ether type of linkage. The maleimide group can for example be introduced into the epitope by reacting the amino group of the epitope with succinimidyl 4-(N-maleimidomethyl)cyclo-hexane-1-carboxylate. In the second step a sulphydryl group containing protein toxin is linked to the maleimide activated epitope.

The carbohydrate moiety of a protein toxin can also be used for linking to the epitope. Periodate is used to generate aldehyde groups into the carbohydrate moiety. Subsequently the aldehyde groups are allowed to react with amino groups of the epitope to form a Schiff's base, which can be stabilised by reduction to give a secondary amine.

Coupling of radio-isotopes to the epitope is achieved by using a chelator. Chelators are constructed in such a way that good in vivo stability of the chelator-isotope complex is assured.

Another way for the construction of immunotoxins according to the present invention is the preparation through recombinant DNA techniques. Relevant nucleic acid sequences derived from genomic DNA or mRNA coding for toxin polypeptides or toxic domains thereof are connected with nucleotide sequences carrying genetic information for the amino acid sequence of the epitope. Optionally, a linking sequence encoding an amino acid sequence which is suitable as a spacer between the toxin and the epitope may be incorporated. Production of these immunotoxins may be achieved in either procaryotic or eucaryotic expression systems.

The immunotoxins of the present invention can be administered enterally or preferably parenterally. For this purpose they are mixed with one or more usual pharmaceutically acceptable non-toxic carriers and/or usual excipients, resulting in a pharmaceutical composition.

Said pharmaceutical composition includes tablets, pills and coated tablets for enteral administration and solutions, suspensions and emulsions for oral or parenteral administration.

The dosage of the compounds in the preparation according to the invention depends greatly on the individual requirement of the patient. However, for intravenous injection the compounds are preferably administered by an initial dosage between 0.1 µg and 1 mg per kg bodyweight, followed if necessary, by one or more supplementary dosages per day. For infusion the compounds are administered during a prolonged time at a dosage which is based on the above-noted intravenous administration.

The present invention is further illustrated by way of the following examples.

Example 1

Synthesis of the nonapeptide Thr-Ala-Val-Val-Ala-Leu-Glu-Leu-Gln-OH (I).

The synthesis of the nonapeptide with the formula I is carried out by the method of solid phase peptide synthesis, as originally described by Merrifield (J.Amer.Chem.Soc. 85, 2149, 1963).

The synthesis is carried out on a Vega Coupler 250 C apparatus, using a p-benzyloxybenzyl alcohol resin (0.6-0.7 mmol/g, Bachem A.G., Switzerland).

Amino acids are coupled to the resin in a stepwise manner as their $N^\alpha$-Fmoc-(Fmoc = 9-fluorenylmethyloxycarbonyl-) derivatives, while the side chain functions of Glu and Thr are protected as a t-butylester and a t-butylether, respectively.

The synthesis starts by the coupling of Fmoc-Gln-OH to the resin, using DCC (dicyclohexylcarbodiimide) and DMAP (N,N-dimethylaminopyridine) at low temperature and in the presence of HOBt (N-hydroxybenzotriazole), as described by Van Nispen et al. (Recl.Trav.Chim. Pays-Bas 104, 99-100, 1985). Residual free alcohol groups on the resulting Fmoc-Gln-resin (0.3-0.4 mmol/g) are blocked by means of acetylation.

The Fmoc-protecting group is removed by treatment of the resin with 25% piperidine in DMF (dimethylformamide) for 10 minutes, followed by 3 washings (one minute each) with DMF and $CH_2Cl_2$.

Fmoc-Leu-OH, Fmoc-Glu(OtBu) -OH, Fmoc-Leu-OH, Fmoc-Ala-OH, Fmoc-Val-OH (2 times), Fmoc-Ala-OH and Fmoc-Thr(tBu)-OH are successively coupled using 3 equivalents each of the protected amino acid, DCC and HOBt in 6-7 ml of DMF per gram of resin for 2 hours. Then 3 washings (one minute each) with ethanol, DMF and $CH_2Cl_2$ are carried out. The completeness of each acylation is monitored by the ninhydrin test of Kaiser et al. (Anal. Biochem. 34, 595-598, 1970). In case of a positive test the pertinent coupling step is repeated with one equivalent each of Fmoc-amino acid, DCC and HOBt for 1 hour. If the Kaiser test is then still positive, the remaining free amino groups are acetylated by a treatment of the resin with acetic anhydride-pyridine (2:1; v/v; 6-7 ml/g) for 10 minutes, followed by 3 washings with DMF and $CH_2Cl_2$ for 1 minute each. Following removal of the Fmoc-protecting group by treatment with 25% piperidine in DMF, as described above, the next amino acid is coupled.

The synthesis results after removal of the final Fmoc-protecting group in the nonapeptide-resin II

$$\underset{\displaystyle \text{H-Thr-Ala-Val-Val-Ala-Leu-Glu-Leu-Gln-}}{\overset{\displaystyle \overset{\textstyle tBu}{\mid}}{\phantom{x}} \qquad\qquad \overset{\displaystyle \overset{\textstyle OtBu}{\mid}}{\phantom{x}}} \boxed{\text{resin}}$$

II

The free peptide I is obtained from nonapeptide-resin II following a treatment in trifluoroacetic acid-dichloromethane (1:1; v/v) for three hours.

This acid treatment simultaneously effects the removal of the t-butyl-based protecting groups and the splitting of the peptide from the resin. The resin is removed by filtration. The filtrate is subsequently evaporated in vacuo. The residue is dissolved in t-butanol-water (1:1; v/v). Remaining TFA is then exchanged for acetic acid by addition of Dowex 2 x 8 (OAc) ion-exchange resin.

Lyophilisation of the resulting solution affords nonapeptide I (as the acetate salt).

## Example 2

### Synthesis of nonapeptide derivatives

#### A. S-acetylthioacetyl derivative

Peptide I is dissolved in DMF-$H_2O$ (2:1; v/v). The pH of the solution is raised to 7.5-8.0 by addition of N-ethyl, N,N-diisopropylamine. A solution of 1.2 equivalents of N-succinimidyl-S-acetylthioacetate (SATA) in DMF is then added. The pH of the reaction mixture is maintained at 7.5-8.0 by addition of said tertiary base. After 30 minutes at room temperature the pH of the solution is brought to 5.0 by adding acetic acid. Following removal of solvents by evaporation in vacuo, the residue is triturated with ethylacetate-ether. The peptide derivative III is subsequently dissolved in t-butanol-water (1:1; v/v) and isolated by lyophilisation.

$$\underset{}{CH_3-\overset{\overset{\textstyle O}{\|}}{C}-S-CH_2-\overset{\overset{\textstyle O}{\|}}{C}-\text{Thr-Ala-Val-Val-Ala-Leu-Glu-Leu-Gln-OH}}$$

III

#### B. Maleimide-derivative

Acylation of the $N^\alpha$-amino group in I, using the procedure described in Example 2A, with N-succinimidyl-4-(N-maleimidomethyl)-cyclohexane-1-carboxylate (SMCC, "Pierce") affords the maleimide-derivative IV.

#### C. Bromoacetyl-derivative

Acylation of the $N^\alpha$-amino group in I, using the procedure described in Example 2A, with N-succinimidylbromoacetate, affords the bromoacetyl-derivative V.

#### D. Pyridyldisulphide-derivative

Acylation of the $N^\alpha$-amino group in I, using the procedure described in Example 2A, with N-succinimidyl-3-(2-pyridyldithio)-propionate (SPDP, "Pierce"), affords the pyridyldisulfide-derivative VI.

## Example 3

### Synthesis of gelonin derivatives

A. Iminothiolane derivative

The free SH-groups are introduced into gelonin by derivatizing the toxin with 2-iminothiolane (Trauts reagent).

A 50-fold molar excess of 2-iminothiolane (0.4 M 2-iminothiolane in 0.1 M phosphate buffer pH 8.0, containing 1 mM EDTA; final concentration 2-iminothiolane is 5 mmol/l) is added to 4 ml gelonin solution (3 mg/ml; 100 μmol/l) in the above-noted buffer. The reaction mixture is incubated for 30 minutes at 30 °C. Subsequently the mixture is applied to a column of Sephadex® G25, equilibrated with 0.1 M phosphate buffer pH 7.5, 0.1 M NaCl, 1 mM EDTA, to remove excess reagent and low molecular weight reaction products. The yield of this derivatization step was 92% and the toxin contained an average of 1-2 SH-groups per gelonin molecule.

B. Pyridyldisulphide-derivative

Pyridyldisulphide (PDP) groups are introduced into gelonin by dissolving gelonin in a 0.1 M phosphate buffer pH 7.5, 0.1 M NaCl, 1 mM EDTA (3 g gelonin/l). A 5 fold molar excess of succinimidyl-pyridyldithio-propionate (40 mM SPDP in ethanol; final concentration SPDP is 500 μmol/l) is added to 4 ml gelonin solution (3 g gelonin/l) in 0.1 M phosphate buffer pH 7.5, 0.1 M NaCl, 1 mM EDTA and incubated 30 minutes at room temperature. Subsequent the mixture is applied to a column of Sephadex® G25 equilibrated in the above-noted buffer to remove excess reagent and low molecular weight reaction products. The recoverage of the toxin was 95% and the toxin molecules contained an average of 1-2 pyridyldisulphide groups per gelonin molecule.

## Example 4

### Linking of the nonapeptide derivative to the gelonin derivative

A. Linking of nonapeptide IV to the gelonin-iminothiolane derivative

8 ml Gelonin derivative solution (1.4 mg/ml) is incubated at room temperature with a 2.5 fold molar excess of nonapeptide IV (final concentration 115 μmol/l) for 2 hours. The incubation buffer is a phosphate buffer pH 7.5, 0.1 M NaCl, 1 mM EDTA. Subsequently the reaction mixture is applied to a Sephadex® G50 column, equilibrated with incubation buffer, to remove excess reagent and low molecular weight reaction products. 72% Gelonin derivative is converted into the conjugate as tested by SDS-PAGE, the main component containing 1 gelonin molecule per peptide.

Free gelonin is removed by ion-exchange chromatography on Mono Q® (0.05 M Tris-buffer, pH 8.0). The conjugate is eluted at 0.5 M NaCl.

B. Linking of nonapeptide VI to the gelonin-iminothiolane derivative.

The preparation of the above-noted conjugate is carried out using the procedure described under A.

C. Linking of nonapeptide V to the gelonin-pyridyldisulphide derivative.

The preparation of the above-noted conjugate is carried out using the procedure described under A.

## Example 5

### Linking of nonapeptide III to the gelonin-pyridyldisulphide derivative

0.5 ml Gelonin derivative (1.4 mg/ml) is incubated overnight at room temperature with a 2.5 fold molar excess of nonapeptide III (final concentration 115 μmol/l) in the above-noted phosphate buffer to which hydroxylamine is added (10 mM).

After the reaction is completed the mixture is applied to a Sephadex® G50 column, equilibrated in incubation buffer, to remove excess reagent and low molecular weight reaction products. 67% Gelonin derivative is converted into the conjugate as tested by SDS-PAGE. Free gelonin is removed by ion-exchange chromatography on Mono Q® (0.05 M Tris-buffer, pH 8.0). The conjugate is eluted at 0.5 M NaCl.

Example 6

Linking an epitope (RdV$_1$) consisting of 20 amino acids of the 65-85 amino acid sequence of the 65 kD protein of Mycobacterium Tuberculosis (MT) to Ricin-A.

The peptide with the sequence Lys-Thr-Ile-Ala-Tyr-Asp-Glu-Glu-Ala-Arg-Arg-Gly-Leu-Glu-Arg-Gly-Ala-Val-Arg-Asn-Ala-Lys is diluted in DMF/H$_2$O (1:2). 0.4 M SPDP in DMF is added (molar ratio 1:1) and the mixture is incubated for 30 min at ambient temperature.

After incubation 2 volumes of ethylacetate are added and the mixture is centrifuged for 5 min in an eppendorf centrifuge at maximum speed. The precipitate is washed with ether and again centrifuged for 5 min at maximum speed. The precipitate is then dried under nitrogen gas.

The dried precipitate is dissolved in 0.1 M NaP (pH = 7.3) in as small a volume as possible.

Ricin-A (in 0.1 M NaP pH = 7.3) is reduced by adding 1 M DTT to a concentration of 10 mM and incubation of the mixture for 30 min at ambient temperature. The solution is then desalted over PD 10 in 0.1 M NaP (pH = 7.3).

The reduced Ricin-A is added to the SPDP-treated peptide in a molar ratio of 1:5. The mixture is incubated at ambient temperature for 1 h.

The resulting conjugate solution may be stored at 4 °C. If necessary, impurities with a high molecular weight can be removed chromatographically (Fractogel HW 55 (s) in PBS).

Example 6B

A humane T-cell clone of an RA patient is stimulated with the 65 kD protein antigen-specific in the presence of Antigen Presenting Cells (APC). Several concentrations of ricin-A, 65 kD Ricin-A or RdV$_1$-ricin-A are added. After three days the proliferation of the cells is measured through the incorporation of 3H-thymidine. The results are represented below as percentages of the incorporation in stimulated cultures without addition of toxin (conjugates).

a and b are a first and a second experiment respectively.

| μg/ml toxin | | Ricin-A % incorporation | | Ricin-A-65 kD % incorporation | | Ricin-A-RdV$_1$ % incorporation |
|---|---|---|---|---|---|---|
| a | b | a | b | a | b | a |
| 0 | (0) | 100 | (100) | 100 | (100) | 100 |
| 0.1 | (0.02) | 71 | (96) | 45 | (86) | 30 |
| 0.3 | (0.2) | 90 | (64) | 32 | (78) | 26 |
| 1.0 | (2.0) | 80 | (22) | 47 | (76) | 24 |
| 3.0 | (20.0) | 24 | (6) | 31 | (59) | 17 |
| 10.0 | | 11 | | 44 | | 18 |
| 30.0 | | 7 | | 19 | | 7 |

The LD$_{50}$ values for the above shown experiments are

|  | Ricin-A | 65 kD-Ricin-A | $RdV_1$-Ricin-A |
|---|---|---|---|
| $LD_{50}$ | 1.1 | 33 | <0.1 |

Therefore we may conclude that the epitope-Ricin-A conjugate is more toxic for these cells than the toxin alone or the toxin-65 kD conjugate.

Example 6C

The same experiments as in example 6B are repeated with the proviso that there are no antigen presenting cells present and that the T-cells are stimulated with IL-2 (antigen non-specific). The results are shown below.

| $\mu$g/ml toxin | | Ricin-A | | Ricin-A-65 kD | | Ricin-A-$RdV_1$ |
|---|---|---|---|---|---|---|
| a | b | a | b | a | b | a |
| 0 | (0) | 100 | (100) | 100 | (100) | 100 |
| 0.1 | (0.02) | 105 | (100) | 96 | (107) | 93 |
| 0.3 | (0.2) | 92 | (93) | 89 | (90) | 90 |
| 1.0 | (2.0) | 85 | (80) | 73 | (81) | 75 |
| 3.0 | (20.0) | 82 | (1) | 31 | (17) | 27 |
| 10.0 | | 47 | | 17 | | 25 |
| 30.0 | | 4 | | 1 | | 2 |

The average $LD_{50}$ values are:

|  | toxin | toxin-65 kD | toxin-$RdV_1$ |
|---|---|---|---|
| average $LD_{50}$ | 6.8 | 3.9 | 1.7 |

It can be concluded that the $RdV_1$-Ricin-A conjugates are the most effective in inhibiting the proliferation of this T-cell clone.

Example 7

In male Lewis-rats adjuvant arthritis is induced by mycobacterium butyricum (100 mg/ml in paraffin-oil).
6 Groups of 5 rats each are treated at day 0-5 and day 8-12 with injections of the following:
group 1   placebo
group 2   65 kD-Ricin-A conjugate (see example 6) (1 mg/kg)
group 3   peptide I-Ricin-A conjugate (see example 5) (0.25 mg/kg)
group 4   Ricin-A (0.25 mg/kg)
group 5   65 kD protein (0.5 mg/kg)
group 6   peptide I (0.01 mg/kg).
On day 22 10 $\mu$g 65 kD-protein is injected into the ear.
On day 24 the swelling of the ear is determined.

The results are averaged per group and represented as percentages of swelling.

| group | % swelling |
|-------|------------|
| (1) | $5.0 \pm 2.6$ |
| (2) | $8.1 \pm 2.8$ |
| (3) | $-0.2 \pm 2.5$ |
| (4) | $8.1 \pm 3.0$ |
| (5) | $5.8 \pm 2.9$ |
| (6) | $2.8 \pm 3.4$ |

It may be concluded that the nonapeptide-Ricin conjugate inhibits the DTH (Delayed Type Hypersensitivity) against 65 kD protein in rats.

## Claims

### Claims for the following Contracting States: AT, BE, CH, DE, FR, GB, IT, LI, NL, SE

1. Immunotoxin for the treatment or prophylaxis of an auto-immune disease, characterized in that the immunotoxin comprises an epitope, recognized by T-lymphocytes being characteristic for the specific auto-immune disease or a cross-reactive analogue thereof, said epitope or cross-reactive analogue thereof being coupled to a cytotoxic substance.

2. Immunotoxin for the treatment or prophylaxis of rheumatoid arthritis according to claim 1, characterized in that the epitope resides in joint cartilage protein.

3. Immunotoxin according to claim 2, characterized in that the epitope has the amino acid sequence Thr-Ala-Val-Val-Ala-Leu-Glu-Leu-Gln.

4. Immunotoxin for the treatment or prophylaxis of rheumatoid arthritis according to claim 1, characterized in that the epitope resides in the 65 kD protein of Mycobacterium.

5. Immunotoxin according to claim 4, characterized in that the epitope has the amino acid sequence Thr-Phe-Gly-Leu-Gln-Leu-Glu-Leu-Thr.

6. Immunotoxin for the treatment or prophylaxis of multiple sclerosis according to claim 1, characterized in that the epitope resides in myelin basic protein.

7. Immunotoxin for the treatment or prophylaxis of myasthenia gravis according to claim 1, characterized in that the epitope resides in the acetylcholine receptor.

8. Immunotoxin according to claims 1-7, characterized in that the cytotoxic substance is a ribosome-inactivating protein.

9. Immunotoxin according to claims 1-7, characterized in that the cytotoxic substance is a radio-isotope.

10. Pharmaceutical preparation containing as biological active components one or more immunotoxins according to claims 1-9.

11. Process for the preparation of immunotoxins according to claim 1-9, characterized in that the epitope or a cross-reactive analogue thereof is coupled to the cytotoxic substance either directly or via a linker.

### Claims for the following Contracting States: ES, GR

1. A process for Manufacturing an immunotoxin for the treatment or prophylaxis of an auto-immune disease, characterized in that an epitope, recognized by T-lymphocytes characteristic of said auto-immune disease, or a cross-reactive analogue of said epitope is coupled to a cytotoxic substance either directly or through a linker.

2. A process according to claim 1, characterized in that an epitope or a cross-reactive analogue thereof, derived from cartillage protein, is coupled to a cytotoxic compound.

3. A process according to claim 2, characterized in that the epitope being coupled comprises the amino acid sequence Thr-Ala-Val-Val-Ala-Leu-Glu-Leu-Gln.

4. A process according to claim 1, characterized in that the epitope is found in the 65kD protein of Mycobacterium.

5. A process according to claim 4, characterized in that the epitope comprises the amino acid sequence Thr-Phe-Gly-Leu-Gln-Leu-Glu-Leu-Thr.

6. A process according to claim 1, characterized in that  the epitope is found in myelin basic protein.

7. A process according to claim 1, characterized in that the epitope is found in the acetylcholine receptor.

8. A process according to any of the aforegoing claims, characterized in that a ribosome-inactivating protein is used as the cytotoxic substance.

9. A process according to any one of the claims 1-7, characterized in that a radio-isotope is used as the cytotoxic substance.

10. A process for manufacturing a pharmaceutical formulation for the treatment or prophylaxis of auto-immune diseases, characterized in that an immunotoxin manufactured according to any one of the aforegoing claims is mixed with suitable excipients.

## Patentansprüche

### Patentansprüche für folgende Vertragsstaaten: AT, BE, CH, DE, FR, GB, IT, LI, LU, NL, SE

1. Immunotoxin für die Behandlung oder die Prophylaxe einer Autoimmunerkrankung, dadurch gekennzeichnet, dass das Immunotoxin ein Epitop enthält, welches durch T-Lymphocyten als charakteristisch für die spezifische Autoimmunerkrankung erkannt wird, oder ein CR-(Cross reactive) Analoges davon enthält, wobei das Epitop oder das CR-Analoge davon an eine cytotoxische Substanz gekoppelt ist.

2. Immunotoxin für die Behandlung oder die Prophylaxe von rheumatoider Arthritis nach Patentanspruch 1, dadurch gekennzeichnet, dass das Epitop in Gelenkknorpelprotein vorhanden ist.

3. Immunotoxin nach Patentanspruch 2, dadurch gekennzeichnet, dass das Epitop die Aminosäuresquenz Thr-Ala-Val-Val-Ala-Leu-Glu-Leu-Gln aufweist.

4. Immunotoxin für die Behandlung oder die Prophylaxe von rheumatoider Arthritis nach Patentanspruch 1, dadurch gekennzeichnet, dass das Epitop im 65 KD-Protein von Mycobakterium enthalten ist.

5. Immunotoxin nach Patentanspruch 4, dadurch gekennzeichnet, dass das Epitop die Aminosäuresquenz Thr-Phe-Gly-Leu-Gln-Leu-Glu-Leu-Thr aufweist.

6. Immunotoxin für die Behandlung oder die Prophylaxe von Multiple Sclerose nach Patentanspruch 1, dadurch gekennzeichnet, dass das Epitop in Myelin-Basisprotein vorhanden ist.

7. Immunotoxin für die Behandlung oder die Prophylaxe von Myasthenia Gravis nach Patentanspruch 1, dadurch gekennzeichnet, dass das Epitop im Acetylcholinerezeptor vorhanden ist.

8. Immunotoxin nach den Patentansprüchen 1 bis 7, dadurch gekennzeichnet, dass die cytotoxische Substanz ein Ribosomen inaktivierendes Protein ist.

9. Immunotoxin nach den Patentansprüchen 1 bis 7, dadurch gekennzeichnet, dass die cytotoxische Substanz ein Radio-Isotop ist.

10. Pharmazeutisches Präparat, enthaltend ein oder mehrere Immunotoxine nach den Patentansprüchen 1 bis 9 als biologisch aktive Komponente.

11. Verfahren zur Herstellung von Immunotoxinen nach den Patentansprüchen 1 bis 9, dadurch gekennzeichnet, dass das Epitop oder ein CR (Cross reactive)-Analoges davon an die cytotoxische Substanz entweder direkt oder über einen Linker gekuppelt wird.

### Patentansprüche für folgende Vertragsstaaten: ES, GR

1. Verfahren zur Herstellung eines Immunotoxins für die Behandlung oder die Prophylaxe einer autoimmunen Erkrankung, dadurch gekennzeichnet, dass ein Epitop, welches durch T-Lymphocyten als charakteristisch für diese autoimmune Erkrankung erkannt wird, oder ein CR (Cross-reactive)-Analoges von diesem Epitop an eine cytotoxische Substanz entweder direkt oder über einen Linker gekoppelt wird.

2. Verfahren nach Patentanspruch 1, dadurch gekennzeichnet, dass ein Epitop oder ein CR (Cross-reactive)-Analoges davon, abgeleitet von Knorpelprotein, an eine cytotoxische Verbindung gekoppelt wird.

3. Verfahren nach Patentanspruch 2, dadurch gekennzeichnet, dass das Epitop, welches gekoppelt wird, die Aminosaüresequenz Thr-Ala-Val-Val-Ala-Leu-Glu-Leu-Gln umfasst.

4. Verfahren nach Patentanspruch 1, dadurch gekennzeichnet, dass das Epitop im 65 KD-Protein von Mycobacterium gefunden wird.

5. Verfahren nach Patentanspruch 4, dadurch gekennzeichnet, dass das Epitop die Aminosäuresequenz Thr-Phe-Gly-Leu-Gln-Leu-Glu-Leu-Thr umfasst.

6. Verfahren nach Patentanspruch 1, dadurch gekennzeichnet, dass das Epitop in Myelin-Basisprotein gefunden wird.

7. Verfahren nach Patentanspruch 1, dadurch gekennzeichnet, dass das Epitop im Acetylcholin-Rezeptor gefunden wird.

8. Verfahren nach einem vorstehenden Patentansprüche, dadurch gekennzeichnet, dass ein Ribosomen inaktivierendes Protein als cytotoxische Substanz verwendet wird.

9. Verfahren nach einem der Patentansprüche 1 bis 7, dadurch gekennzeichnet, dass ein Radio-Isotop als cytotoxische Substanz verwendet wird.

10. Verfahren zur Herstellung eines pharmazeutischen Präparates für die Behandlung oder Prophylaxe von autoimmunen Erkrankungen, dadurch gekennzeichnet, dass ein gemäss einem der vorstehenden Patentansprüche hergestelltes Immunotoxin mit geeigneten Excipienten vermischt wird.

## Revendications

**Revendications pour les Etats contractants suivants: AT, BE, CH, DE, FR, GB, IT, LI, LU, ML, SE**

1. Immunotoxine pour le traitement ou la prophylaxie d'une maladie auto-immune, caractérisée par le fait que l'immunotoxine comprend un épitope, reconnu par les lymphocytes T caractéristiques de la maladie auto-immune particulière, ou un analogue à réaction croisée de celui-ci, ledit épitope ou son analogue à réaction croisée étant couplé à une substance cytotoxique.

2. Immunotoxine pour le traitement ou la prophylaxie de la polyarthrite rhumatoïde selon la revendication 1, caractérisée par le fait que l'épitope se trouve dans une protéine de cartilage articulaire.

3. Immunotoxine selon la revendication 2, caractérisée par le fait que l'épitope possède la séquence d'acides aminés Thr-Ala-Val-Val-Ala-Leu-Glu-Leu-Gln.

4. Immunotoxine pour le traitement ou la prophylaxie de la polyarthrite rhumatoïde selon la revendication 1, caractérisée par le fait que l'épitope se trouve dans la protéine de 65 kd de Mycobacterium.

5. Immunotoxine selon la revendication 4, caractérisée par le fait que l'épitope possède la séquence d'acides aminés Thr-Phe-Gly-Leu-Gln-Leu-Glu-Leu-Thr.

6. Immunotoxine pour le traitement ou la prophylaxie de la sclérose en plaques selon la revendication 1, caractérisée par le fait que l'épitope se trouve dans la protéine de base de la myéline.

7. Immunotoxine pour le traitement ou la prophylaxie de la myasthénie selon la revendication 1, caractérisée par le fait que l'épitope se trouve dans le récepteur d'acétylcholine.

8. Immunotoxine selon les revendications 1 à 7, caractérisée par le fait que la substance cytotoxique est une protéine inactivant les ribosomes.

9. Immunotoxine selon les revendications 1 à 7, caractérisée par le fait que la substance cytotoxique est un radio-isotope.

10. Formulation pharmaceutique contenant comme composants biologiquement actifs une ou plusieurs immunotoxines selon les revendications 1 à 9.

11. Procédé pour la préparation d'immunotoxines selon les revendications 1 à 9, caractérisé par le fait que l'épitope ou son analogue à réaction croisée est couplé à la substance cytotoxique, soit directement, soit par l'intermédiaire d'une molécule de liaison.

**Revendications pour les Etats contractants suivants: ES, GR**

1. Procédé de préparation d'une immunotoxine pour le traitement ou la prophylaxie d'une maladie auto-immune, caractérisé par le fait qu'un épitope, reconnu par les lymphocytes T caractéristiques de ladite maladie autoimmune, ou un analogue à réaction croisée dudit épitope est couplé à une substance cytotoxique, soit directement, soit par l'intermédiaire d'une molécule de liaison.

2. Procédé selon la revendication 1, caractérisé par le fait qu'un épitope ou un analogue à réaction croisée de celui-ci, dérivé de protéine de cartilage, est couplé à un composé cytotoxique.

3. Procédé selon la revendication 2, caractérisé par le fait que l'épitope qui est couplé comprend la séquence d'acides aminés Thr-Ala-Val-Val-Ala-Leu-Glu-Leu-Gln.

4. Procédé selon la revendication 1, caractérisé par le fait que l'épitope se trouve dans la protéine de 65 kd de Mycobacterium.

5. Procédé selon la revendication 4, caractérisé par le fait que l'épitope comprend la séquence d'acides aminés Thr-Phe-Gly-Leu-Gln-Leu-Glu-Leu-Thr.

6. Procédé selon la revendication 1, caractérisé par le fait que l'épitope se trouve dans la protéine de base de la myéline.

7. Procédé selon la revendication 1, caractérisé par le fait que l'épitope se trouve dans le récepteur d'acétylcholine.

8. Procédé selon l'une quelconque des revendications précédentes, caractérisé par le fait qu'une protéine inactivant les ribosomes est utilisée comme substance cytotoxique.

9. Procédé selon l'une quelconque des revendications 1 à 7, caractérisé par le fait qu'un radio-isotope est utilisé comme substance cytotoxique.

10. Procédé de fabrication d'une formulation pharmaceutique pour le traitement ou la prophylaxie de maladies auto-immunes, caractérisé par le fait qu'une immunotoxine préparée selon l'une quelconque des revendications précédentes est mélangée avec des excipients appropriés.